# FASCICULE DE BREVET EUROPEEN

(11) **EP 3 247 748 B1**
(45) Date de publication et mention de la délivrance du brevet: **22.05.2024**
(21) Numéro de dépôt: 16701120.4
(22) Date de dépôt: 20.01.2016
(51) Int. Cl.: C08L 61/12, C08G 8/06, C08L 7/00, C08L 9/00, C08G 8/04, B60C 1/00

(54) **COMPOSITION DE CAOUTCHOUC À HAUTE RIGIDITÉ**
HOCHSTEIFE KAUTSCHUKZUSAMMENSETZUNG
HIGH-RIGIDITY RUBBER COMPOSITION

(30) Priorité: 21.01.2015 FR 1550456
(43) Date de publication de la demande: 29.11.2017
(73) Titulaire: Compagnie Générale des Etablissements Michelin, 63000 Clermont-Ferrand (FR)
(72) Inventeur: DOISNEAU, David, 63040 Clermont-Ferrand Cedex 9 (FR); THUILLIEZ, Anne-Lise, 63040 Clermont-Ferrand Cedex 9 (FR); GAVARD-LONCHAY, Odile, 63040 Clermont-Ferrand Cedex 9 (FR)
(74) Mandataire: M.F.P. Michelin
(86) Numéro de dépôt international: PCT/EP2016/051051
(87) Numéro de publication internationale: WO 2016/116468

(56) Documents cités:
- WO-A1-2015/007642
- WO-A1-2015/118042
- US-A- 5 030 692
- US-A1- 2012 101 211
- US-A1- 2012 211 139

## Description

L'invention concerne une composition de caoutchouc, un procédé de fabrication de cette composition, un composite de caoutchouc et un pneumatique.

Il est connu d'utiliser dans certaines parties des pneumatiques, des compositions de caoutchouc présentant une forte rigidité lors de faibles déformations du pneumatique. La résistance aux faibles déformations est une des propriétés que doit présenter un pneumatique pour répondre aux sollicitations auxquelles il est soumis.

Une forte rigidité peut être obtenue en utilisant un système de vulcanisation dit concentré, c'est-à-dire comprenant notamment des taux de soufre et d'accélérateur de vulcanisation relativement élevés.

Toutefois, un tel système de vulcanisation concentré pénalise le vieillissement à cru de la composition. Ainsi, lorsque la composition est sous forme de produit semi-fini, par exemple de bande de gomme, le soufre peut migrer en surface du produit semi-fini. Ce phénomène, appelé effleurissement, entraine une pénalisation du collant à cru du produit semi-fini lors de son stockage prolongé, avec pour conséquence une dégradation de l'adhésion entre les produits semi-finis lors de la fabrication du pneumatique.

Par ailleurs, le stockage de la composition crue contenant un système de vulcanisation concentré est susceptible d'entrainer une diminution de la phase retard de la composition lors de sa vulcanisation, c'est-à-dire le temps précédant le début de la vulcanisation. En conséquence, la composition peut commencer à cuire prématurément dans certains outils de mise en forme et la cinétique de vulcanisation est susceptible d'être modifiée et le rendement de vulcanisation d'être dégradé.

Un tel système de vulcanisation concentré pénalise également le vieillissement à cuit. En effet, on observe une dégradation des propriétés mécaniques de la composition cuite, notamment aux limites, par exemple de l'allongement à rupture.

Une forte rigidité peut autrement être obtenue en augmentant le taux de charge renforçante.

Toutefois, de manière connue, l'augmentation de la rigidité d'une composition de caoutchouc en augmentant le taux de charge peut pénaliser les propriétés d'hystérèse et donc de résistance au roulement des pneumatiques. Or, on cherche toujours à baisser la résistance au roulement des pneumatiques pour diminuer la consommation en carburant et ainsi préserver l'environnement.

Enfin, une rigidité élevée peut être obtenue en incorporant certaines résines renforçantes comme divulgué dans WO 02/10269.

Classiquement l'augmentation de la rigidité est obtenue en incorporant des résines renforçantes à base d'un système accepteur/donneur de méthylène. Les termes « accepteur de méthylène » et « donneur de méthylène » sont bien connus de l'homme du métier et largement utilisés pour designer des composés aptes à réagir ensemble pour générer par condensation une résine renforçante tridimensionnelle qui vient se superposer et s'interpénétrer avec le réseau charge renforçante/élastomère d'une part et, avec le réseau élastomère/soufre d'autre part (si l'agent de réticulation est le soufre). A l'accepteur de méthylène est associé un agent durcisseur, apte à le réticuler ou le durcir, encore appelé communément "donneur de méthylène". Des exemples de tels accepteur et donneur de méthylène sont décrits dans WO 02/10269.

Les donneurs de méthylène classiquement utilisés dans les compositions de caoutchouc pour pneumatiques sont l'hexa-méthylènetétramine (en abrégé HMT), ou l'hexamethoxyméthylmélamine (en abrégé HMMM ou H3M), ou l'hexaethoxyméthylmélamine.

Les accepteurs de méthylène classiquement utilisés dans les compositions de caoutchouc pour pneumatiques sont des résines phénoliques précondensées.

Des exemples d'accepteur et de donneur de méthylène sont notamment décrits dans US 5 030 692 et US 2012/101211.

Toutefois, la combinaison de résine phénolique classiquement utilisée en tant qu'accepteur de méthylène avec l'HMT ou l'H3M en tant que donneur de méthylène, produit du formaldéhyde au cours de la vulcanisation de la composition de caoutchouc. Or, il est souhaitable de diminuer, voire de supprimer à terme le formaldéhyde des compositions de caoutchouc en raison de l'impact environnemental de ces composés et de l'évolution récente de la réglementation, notamment la règlementation européenne, sur ce type de composé.

L'invention a pour objet une composition de caoutchouc rigidifiée au moyen de composés à faible impact environnemental, la rigidité étant maintenue dans une plage de températures élevées d'utilisation de la composition de caoutchouc, en particulier pour des températures allant jusqu'à 150°C.

A cet effet, l'invention a pour objet une composition de caoutchouc comprenant au moins une résine phénol-aldéhyde à base:
- d'au moins un polyphénol aromatique comprenant au moins un noyau aromatique porteur d'au moins deux fonctions hydroxyle en position méta l'une par rapport à l'autre, les deux positions ortho d'au moins une des fonctions hydroxyle étant non substituées, et
   - d'au moins un aldéhyde de formule **(A)** : 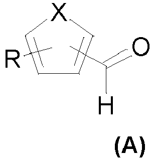
dans laquelle :
X comprend N, S ou O
R représente -H ou -CHO.

De manière inattendue, les Demanderesses ont découvert lors de leurs recherches que les aldéhydes de formule **(A)** de la composition selon l'invention permettent d'éviter la production de formaldéhyde contrairement aux donneurs de méthylène classiques.

En outre, la combinaison spécifique des aldéhydes de formule **(A)** et des polyphénols aromatiques de la composition selon l'invention permet d'obtenir des compositions de caoutchouc présentant une rigidité à basse déformation largement améliorée par rapport aux compositions de caoutchouc classiques qui comprennent des donneurs de méthylène HMT ou H3M.

De plus, la combinaison spécifique des aldéhydes de formule **(A)** et des polyphénols aromatiques de la composition selon l'invention permet de maintenir une rigidité élevée avec l'augmentation de la température.

Enfin, de tels aldéhydes sont issus de ressources renouvelables et pas du pétrole. Les aldéhydes sont par exemple issus de la bio-ressource ou de produits de transformation de la bio-ressource.

Par l'expression « résine à base de », il faut bien entendu comprendre une résine comportant le mélange et/ou le produit de la réaction entre les différents constituants de base utilisés pour cette résine, de préférence uniquement le produit de la réaction entre les différents constituants de base utilisés pour cette résine, certains d'entre eux pouvant être destinés à réagir ou susceptibles de réagir entre eux ou avec leur environnement chimique proche, au moins en partie, lors des différentes phases du procédé de fabrication de la composition, des composites ou du pneumatique, en particulier au cours d'une étape de cuisson.

Par "position méta l'une par rapport à l'autre", on entendra que les fonctions hydroxyle sont portées par des carbones du noyau aromatique séparés l'un de l'autre par un unique autre carbone du noyau aromatique.

Par "en position ortho d'une fonction", on entendra la position occupée par le carbone du noyau aromatique immédiatement adjacent au carbone du noyau aromatique portant la fonction.

La composition de caoutchouc comprend donc au moins une (c'est-à-dire une ou plusieurs) résine renforçante réticulée, cette résine renforçante étant constituée par la résine phénol-aldéhyde; cette résine phénol-aldéhyde étant à base d'au moins un (c'est-à-dire un ou plusieurs) aldéhyde de formule **(A)** et au moins un (c'est-à-dire un ou plusieurs) polyphénol aromatique, constituants qui vont être décrits en détail ci-après.

Un autre objet de l'invention est un procédé de fabrication d'une composition de caoutchouc comprenant une étape de mélangeage :
- d'au moins un élastomère,
- d'au moins un polyphénol aromatique comprenant au moins un noyau aromatique porteur d'au moins deux fonctions hydroxyle en position méta l'une par rapport à l'autre, les deux positions ortho d'au moins une des fonctions hydroxyle étant non substituées, et
- d'au moins un aldéhyde de formule **(A)** : 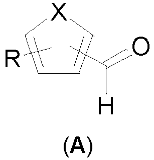 dans laquelle :
   X comprend N, S ou O
   R représente -H ou -CHO.

L'invention a aussi pour objet un composite de caoutchouc renforcé d'au moins un élément de renfort noyé dans une composition de caoutchouc telle que décrite ci-dessus.

Un autre objet de l'invention est un pneumatique comprenant une composition de caoutchouc telle que décrite ci-dessus ou un composite de caoutchouc tel que décrit ci-dessus.

Par composition de caoutchouc, on entend que la composition comprend au moins un élastomère ou un caoutchouc (les deux termes étant synonymes) et au moins un autre composant. Une composition de caoutchouc comprend donc une matrice d'élastomère ou de caoutchouc dans laquelle est dispersé au moins l'autre composant. Une composition de caoutchouc est dans un état plastique à l'état cru (non-réticulé) et dans un état élastique à l'état cuit (réticulé) mais en aucun cas dans un état liquide. Une composition de caoutchouc ne doit pas être confondue avec un latex d'élastomère qui est une composition dans un état liquide comprenant un solvant liquide, généralement de l'eau, et au moins un élastomère ou un caoutchouc dispersé dans le solvant liquide de façon à former une émulsion. Ainsi, la composition de caoutchouc n'est pas une composition adhésive aqueuse.

Dans la présente description, sauf indication expresse différente, tous les pourcentages (%) indiqués sont des pourcentages en poids. Le sigle « pce » signifie parties en poids pour cent parties d'élastomère.

D'autre part, tout intervalle de valeurs désigné par l'expression « entre a et b » représente le domaine de valeurs allant de plus de a à moins de b (c'est-à-dire bornes a et b exclues) tandis que tout intervalle de valeurs désigné par l'expression « de a à b » signifie le domaine de valeurs allant de la borne « a » jusqu'à la borne « b » c'est-à-dire incluant les bornes strictes « a » et « b ».

### Polyphénol aromatique de la composition de caoutchouc

Dans un mode de réalisation préféré, le noyau aromatique du polyphénol aromatique est porteur de trois fonctions hydroxyle en position méta les unes par rapport aux autres.

De préférence, les deux positions ortho de chaque fonction hydroxyle sont non substituées. On entend par là que les deux atomes de carbone situés de part et d'autre (en position ortho) de l'atome de carbone hydroxylé (i.e., porteur de la fonction hydroxyle) sont porteurs d'un simple atome d'hydrogène.

Encore plus préférentiellement, le reste du noyau aromatique du polyphénol aromatique est non substitué. On entend par là que les autres atomes de carbone du reste du noyau aromatique (ceux autres que les atomes de carbone porteurs des fonctions hydroxyle) sont porteurs d'un simple atome d'hydrogène.

Dans un mode de réalisation, le polyphénol aromatique comprend plusieurs noyaux aromatiques, au moins deux d'entre eux étant chacun porteur d'au moins deux fonctions hydroxyle en position méta l'une par rapport à l'autre, les deux positions ortho d'au moins une des fonctions hydroxyle d'au moins un noyau aromatique étant non substituées.

Dans un mode de réalisation préféré, au moins un des noyaux aromatiques du polyphénol aromatique est porteur de trois fonctions hydroxyle en position méta les unes par rapport aux autres.

De préférence, les deux positions ortho de chaque fonction hydroxyle d'au moins un noyau aromatique sont non substituées.

Encore plus préférentiellement, les deux positions ortho de chaque fonction hydroxyle de chaque noyau aromatique sont non substituées.

Avantageusement, le ou chaque noyau aromatique du polyphénol aromatique est un noyau benzénique.

A titre d'exemple de polyphénol aromatique comportant un seul noyau aromatique, on peut citer en particulier, le résorcinol et le phloroglucinol, pour rappel de formules développées :

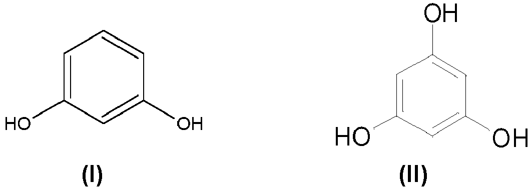

A titres d'exemples, dans le cas où le polyphénol aromatique comporte plusieurs noyaux aromatiques, au moins deux de ces noyaux aromatiques, identiques ou différents, sont choisis parmi ceux de formules générales : 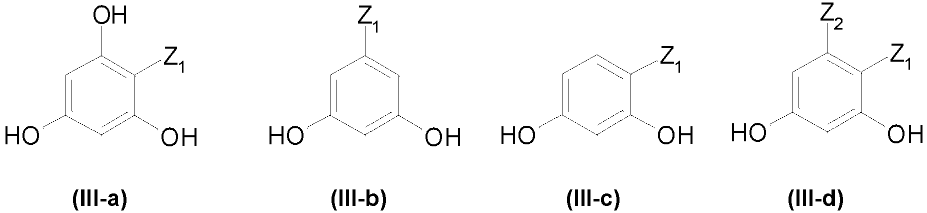 dans lesquelles les symboles Z₁, Z₂, identiques ou différents s'ils sont plusieurs sur le même noyau aromatique, représentent un atome (par exemple carbone, soufre ou oxygène) ou un groupe de liaison par définition au moins divalent, qui relie au moins ces deux noyaux aromatiques au reste du polyphénol aromatique.

Un autre exemple de polyphénol aromatique est le 2,2',4,4'-tétrahydroxydiphényl sulfide de formule développée suivante :

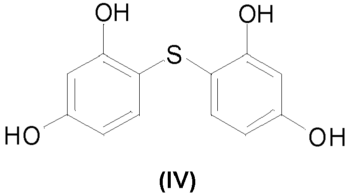

Un autre exemple de polyphénol aromatique est le 2,2',4,4'-tétrahydroxydiphényl benzophénone de formule développée suivante :

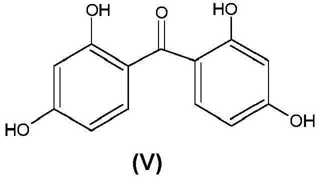

On note que chaque composé **IV** et **V** est un polyphénol aromatique comportant deux noyaux aromatiques (de formules **III-c)** dont chacun est porteur d'au moins deux (en l'occurrence de deux) fonctions hydroxyle en position méta l'une par rapport à l'autre.

On note que dans le cas d'un polyphénol aromatique comportant au moins un noyau aromatique conforme à la formule **III-b,** les deux positions ortho de chaque fonction hydroxyle d'au moins un noyau aromatique sont non substituées. Dans le cas d'un polyphénol aromatique comportant plusieurs noyaux aromatiques conformes à la formule **III-b,** les deux positions ortho de chaque fonction hydroxyle de chaque noyau aromatique sont non substituées.

Selon un mode de réalisation de l'invention, le polyphénol aromatique est choisi dans le groupe constitué par le résorcinol **(I),** le phloroglucinol **(II),** le 2,2',4,4'-tétrahydroxydiphényl sulfide **(IV),** la 2,2',4,4'-tétrahydroxybenzophenone **(V),** les résines pré-condensées à partir d'au moins un de ces phénols et les mélanges de ces composés. Dans un mode de réalisation particulièrement avantageux, le polyphénol aromatique est le phloroglucinol.

Dans le mode de réalisation dans lequel le polyphénol aromatique est une résine pré-condensée à partir d'au moins un de ces phénols, la résine comporte de préférence un motif répétitif, ce motif comprenant un noyau aromatique porteur d'au moins deux fonctions hydroxyle en position méta l'une par rapport à l'autre. Dans le cas où cette résine ne comporte pas de motif répétitif, la résine comporte au moins un noyau aromatique porteur d'au moins deux fonctions hydroxyle en position méta l'une par rapport à l'autre.

### Aldéhyde de formule (A) de la composition de caoutchouc

Préférentiellement, l'aldéhyde est de formule générale **(A'):**

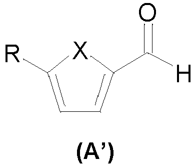

Encore plus préférentiellement, R représente -CHO.

Selon un mode de réalisation préférentiel, X représente O.

Dans une variante de l'aldéhyde de formule générale **(A),** X représente O et R représente -H. L'aldéhyde utilisé est alors de formule **(Ba):**

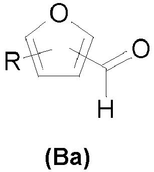

Dans une variante de l'aldéhyde de formule générale **(A'),** X représente O et R représente -H. L'aldéhyde utilisé est alors le furfuraldéhyde et est de formule **(B'a):**

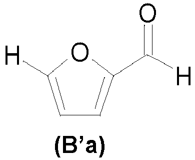

Dans une autre variante de l'aldéhyde de formule générale **(A),** X représente O et R représente -CHO. L'aldéhyde utilisé est alors de formule **(Bb):**

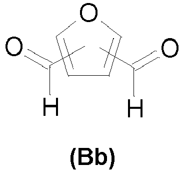

Dans une autre variante de l'aldéhyde de formule générale **(A'),** X représente O et R représente -CHO. L'aldéhyde utilisé est alors le 2,5-furanedicarboxaldéhyde et est de formule **(B'b):**

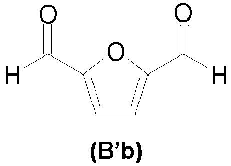

Dans un autre mode de réalisation, X comprend N.

Dans une variante de l'aldéhyde de formule générale **(A),** X représente NH. L'aldéhyde utilisé est de formule **(Ca):**

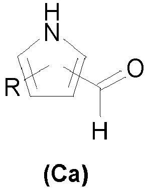

Dans une variante de l'aldéhyde de formule générale **(A'),** X représente NH. L'aldéhyde utilisé est de formule **(C'a):**

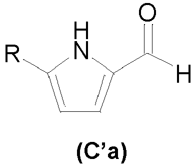

De préférence, R représente -CHO dans la variante de l'aldéhyde de formule **(C'a)** et l'aldéhyde obtenu est alors le 2,5-1H-pyrroledicarboxaldéhyde.

Dans une autre variante de l'aldéhyde de formule générale **(A),** X représente NR1 avec R1 représentant un groupe alkyle, aryle arylalkyle, alkylaryle ou cycloalkyle. L'aldéhyde utilisé est de formule **(Cb):**

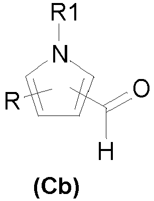

Dans un autre mode de réalisation, X comprend S.

Dans une variante de l'aldéhyde de formule générale **(A),** X représente S. L'aldéhyde utilisé est de formule **(Da):**

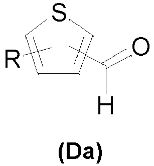

Dans une variante de l'aldéhyde de formule générale **(A'),** X représente S. L'aldéhyde utilisé est de formule **(D'a):**

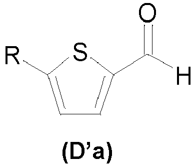

De préférence, R représente -CHO dans la variante de l'aldéhyde de formule **(IV'a)** et est alors le 2,5-thiophènedicarboxaldéhyde.

Dans une autre variante de l'aldéhyde de formule générale **(A),** X représente SR2 avec R2 représentant un groupe alkyle, aryle arylalkyle, alkylaryle ou cycloalkyle. L'aldéhyde utilisé est de formule **(Db):**

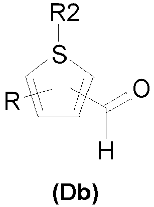

Dans encore une autre variante de l'aldéhyde de formule générale **(A),** X représente R3-S-R2 avec R2, R3 représentant chacun indépendamment l'un de l'autre un groupe alkyle, aryle arylalkyle, alkylaryle ou cycloalkyle. L'aldéhyde utilisé est de formule (De):

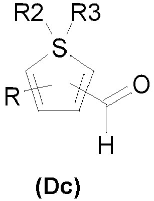

Dans encore une autre variante de l'aldéhyde de formule générale **(A),** X représente S=O. L'aldéhyde utilisé est de formule **(Dd):**

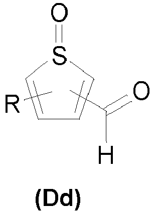

Dans encore une autre variante de l'aldéhyde de formule générale **(A),** X représente O=S=O. L'aldéhyde utilisé est de formule **(De):**

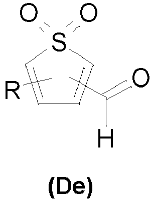

Parmi les différents modes de réalisation décrits ci-dessus, on préférera les modes de réalisation et les variantes dans lesquels X représente NH, S ou O. Dans ces modes de réalisation et variantes, on pourra avoir, conformément à l'invention, R représentant -H ou - CHO et de préférence R représentant -CHO. Dans ces modes de réalisation et variantes, on aura préférentiellement R en position 5 et le groupe -CHO en position 2 sur le noyau aromatique (formule générale **(A')).**

De préférence, la composition est dépourvue de formaldéhyde.

Lorsque la résine phénol-aldéhyde est à base de plusieurs aldéhydes dont l'un au moins est un aldéhyde de formule **(A)** conforme à l'invention, chaque aldéhyde autre que l'aldéhyde de formule **(A)** conforme à l'invention est préférentiellement différent du formaldéhyde. La composition est alors également préférentiellement dépourvue de formaldéhyde.

En d'autres termes et de manière préférée, le ou chaque aldéhyde de la résine phénol-aldéhyde est différent du formaldéhyde.

Par dépourvue de formaldéhyde, on entend que le taux massique de formaldéhyde en poids total du ou des aldéhydes est strictement inférieur à 1%.

Dans certains modes de réalisation, la composition peut comprendre du formaldéhyde. De préférence, la composition comprend alors un taux massique de formaldéhyde en poids total du ou des aldéhydes inférieur ou égal à 10%, de préférence à 5% et plus préférentiellement à 2%.

### Composition de caoutchouc

Dans certains modes de réalisation, on utilisera une quantité d'aldéhyde de formule (A) allant de 0,1 à 30 pce. De même, on utilisera une quantité de polyphénol aromatique allant de 0,1 à 30 pce.

Dans ces modes de réalisation, la composition de caoutchouc présente, à l'état cuit, un module sécant à 10% d'allongement MA10 mesuré selon la norme ASTM D 412 de 1998 (éprouvette C) allant de 5 à 25 MPa, de préférence supérieur ou égal à 25 MPa. Dans des modes de réalisation particulièrement préférés, le MA10 est supérieur ou égal à 30 MPa, plus préférentiellement supérieur ou égal à 35 MPa et encore plus préférentiellement strictement supérieur à 39 MPa.

De préférence, la composition de caoutchouc comprend un élastomère diénique.

Par élastomère ou caoutchouc (les deux termes étant synonymes) du type "diénique", on entend de manière générale un élastomère issu au moins en partie (i.e. un homopolymère ou un copolymère) de monomères diènes (monomères porteurs de deux doubles liaisons carbone-carbone, conjuguées ou non).

De manière particulièrement préférentielle, l'élastomère diénique de la composition de caoutchouc est choisi dans le groupe constitué par les polybutadiènes (BR), les polyisoprènes de synthèse (IR), le caoutchouc naturel (NR), les copolymères de butadiène, les copolymères d'isoprène et les mélanges de ces élastomères. De tels copolymères sont plus préférentiellement choisis dans le groupe constitué par les copolymères de butadiène-styrène (SBR), les copolymères d'isoprène-butadiène (BIR), les copolymères d'isoprène-styrène (SIR), les copolymères d'isoprène-butadiène-styrène (SBIR) et les mélanges de tels copolymères.

Les compositions de caoutchouc peuvent contenir un seul élastomère diénique ou un mélange de plusieurs élastomères diéniques, le ou les élastomères diéniques pouvant être utilisés en association avec tout type d'élastomère synthétique autre que diénique, voire avec des polymères autres que des élastomères, par exemple des polymères thermoplastiques.

De préférence, la composition de caoutchouc comprend une charge renforçante.

Lorsqu'une charge renforçante est utilisée, on peut utiliser tout type de charge renforçante connue pour ses capacités à renforcer une composition de caoutchouc utilisable pour la fabrication de pneumatiques, par exemple une charge organique telle que du noir de carbone, une charge inorganique renforçante telle que de la silice, ou encore un coupage de ces deux types de charge, notamment un coupage de noir de carbone et de silice.

Comme noirs de carbone conviennent tous les noirs de carbone conventionnellement utilisés dans les pneumatiques (noirs dits de grade pneumatique). Par exemple, on citera plus particulièrement les noirs de carbone renforçants des séries 100, 200 ou 300 (grades ASTM).

Dans le cas d'une utilisation de noirs de carbone avec un élastomère isoprénique, les noirs de carbone pourraient être par exemple déjà incorporés à l'élastomère isoprénique sous la forme d'un masterbatch (voir par exemple demandes WO 97/36724 ou WO 99/16600).

Comme exemples de charges organiques autres que des noirs de carbone, on peut citer les charges organiques de polyvinylaromatique fonctionnalisé telles que décrites dans les demandes WO-A-2006/069792 et WO-A-2006/069793.

Par "charge inorganique renforçante", doit être entendu dans la présente demande, par définition, toute charge inorganique ou minérale (quelles que soient sa couleur et son origine (naturelle ou de synthèse), encore appelée charge "blanche", charge "claire" voire "charge non noire" ("non-black filler") par opposition au noir de carbone, capable de renforcer à elle seule, sans autre moyen qu'un agent de couplage intermédiaire, une composition de caoutchouc destinée à la fabrication de pneumatiques, en d'autres termes apte à remplacer, dans sa fonction de renforcement, un noir de carbone conventionnel de grade pneumatique. Une telle charge se caractérise généralement, de manière connue, par la présence de groupes hydroxyle (-OH) à sa surface.

L'état physique sous lequel se présente la charge inorganique renforçante est indifférent, que ce soit sous forme de poudre, de micro-perles, de granules, de billes ou toute autre forme densifiée appropriée. Bien entendu on entend également par charge inorganique renforçante des mélanges de différentes charges inorganiques renforçantes, en particulier de charges siliceuses et/ou alumineuses hautement dispersibles telles que décrites ci-après.

Comme charges inorganiques renforçantes conviennent notamment des charges minérales du type siliceuse, en particulier de la silice (SiO₂), ou du type alumineuse, en particulier de l'alumine (Al₂O₃). La silice utilisée peut être toute silice renforçante connue de l'homme du métier, notamment toute silice précipitée ou pyrogenée présentant une surface BET ainsi qu'une surface spécifique CTAB toutes deux inferieures à 450 m2/g, de préférence de 30 à 400 m2/g. A titres de silices précipitées hautement dispersibles (dites "HDS"), on citera par exemple les silices "Ultrasil" 7000 et "Ultrasil" 7005 de la société Evonik, les silices "Zeosil" 1165MP, 1135MP et 1115MP de la société Rhodia, la silice "Hi-Sil" EZ150G de la société PPG, les silices "Zeopol" 8715, 8745 et 8755 de la Société Huber, les silices à haute surface spécifique telles que décrites dans la demande WO 03/16837.

Enfin, l'homme du métier comprendra qu'à titre de charge équivalente de la charge inorganique renforçante décrite dans le présent paragraphe, pourrait être utilisée une charge renforçante d'une autre nature, notamment organique, dès lors que cette charge renforçante serait recouverte d'une couche inorganique telle que de la silice, ou bien comporterait à sa surface des sites fonctionnels, notamment hydroxyle, nécessitant l'utilisation d'un agent de couplage pour établir la liaison entre la charge et l'élastomère.

De préférence, le taux de charge renforçante totale (noir de carbone et/ou charge inorganique renforçante telle que silice) est compris dans un domaine allant de 5 à 120 pce, plus préférentiellement de 5 à 100 pce et encore plus préférentiellement de 5 à 90 pce.

Préférentiellement, le taux de charge renforçante totale est compris dans un domaine allant de 10 à 120 pce, plus préférentiellement de 10 à 100 pce et encore plus préférentiellement de 10 à 90 pce.

Plus préférentiellement, le taux de charge renforçante totale est compris dans un domaine allant de 20 à 120 pce, plus préférentiellement de 20 à 100 pce et encore plus préférentiellement de 20 à 90 pce.

Encore plus préférentiellement, le taux de charge renforçante totale est compris dans un domaine allant de 30 à 120 pce, plus préférentiellement de 30 à 100 pce et encore plus préférentiellement de 30 à 90 pce.

Le noir de carbone peut avantageusement constituer la seule charge renforçante ou la charge renforçante majoritaire. Bien entendu on peut utiliser un seul noir de carbone ou un coupage de plusieurs noirs de carbone de grades ASTM différents. Le noir de carbone peut être également utilisé en coupage avec d'autres charges renforçantes et en particulier des charges inorganiques renforçantes telles que décrites précédemment, et en particulier de la silice.

Lorsqu'une charge inorganique (par exemple de la silice) est utilisée dans la composition de caoutchouc, seule ou en coupage avec du noir de carbone, son taux est compris dans un domaine de 0 à 70 pce, préférentiellement de 0 à 50 pce, en particulier également de 5 à 70 pce, et encore plus préférentiellement cette proportion varie de 5 à 50 pce, particulièrement de 5 à 40 pce.

De préférence, la composition de caoutchouc comprend des additifs divers.

Les compositions de caoutchouc peuvent comporter également tout ou partie des additifs usuels habituellement utilisés dans les compositions d'élastomères destinées à la fabrication de pneumatiques, comme par exemple des plastifiants ou des huiles d'extension, que ces derniers soient de nature aromatique ou non-aromatique, des pigments, des agents de protection tels que cires anti-ozone, anti-ozonants chimiques, anti-oxydants, des agents anti-fatigue ou bien encore des promoteurs d'adhésion.

De préférence, la composition de caoutchouc comprend un système de réticulation, plus préférentiellement de vulcanisation.

Le système de vulcanisation, comprend un agent donneur de soufre, par exemple du soufre.

De préférence, le système de vulcanisation comprend des activateurs de vulcanisation tels que l'oxyde de zinc et l'acide stéarique.

De préférence, le système de vulcanisation comprend un accélérateur de vulcanisation et/ou un retardateur de vulcanisation.

Le soufre ou agent donneur de soufre est utilisé à un taux préférentiel compris dans un domaine de 0,5 à 10 pce, plus préférentiellement compris dans un domaine de 0,5 à 8,0 pce. L'ensemble des accélérateurs, retardateurs et activateurs de vulcanisation est utilisé à un taux préférentiel compris dans un domaine de 0,5 à 15 pce. Le ou les activateurs de vulcanisation est ou sont utilisés à un taux préférentiel compris dans un domaine de 0,5 et 12 pce.

Le système de réticulation proprement dit est préférentiellement à base de soufre et d'un accélérateur primaire de vulcanisation, en particulier d'un accélérateur du type sulfénamide. A ce système de vulcanisation viennent s'ajouter, divers accélérateurs secondaires ou activateurs de vulcanisation connus tels qu'oxyde de zinc, acide stéarique, dérivés guanidiques (en particulier diphenylguanidine), etc.

On peut utiliser comme accélérateur (primaire ou secondaire) tout composé susceptible d'agir comme accélérateur de vulcanisation des élastomères diéniques en présence de soufre, notamment des accélérateurs du type thiazole ainsi que leurs dérivés, des accélérateurs de type thiurame, et de type dithiocarbamate de zinc. Ces accélérateurs sont plus préférentiellement choisis dans le groupe constitue par le disulfure de 2-mercaptobenzothiazyle (en abrégé "MBTS"), N-cyclohexyl-2-benzothiazyle sulfénamide (en abrégé "CBS"), N,N-dicyclohexyl- 2-benzothiazyle sulfénamide (en abrégé "DCBS"), N-ter-butyl-2-benzothiazyle sulfénamide (en abrégé "TBBS"), N-ter-butyl-2-benzothiazyle sulfénimide (en abrégé "TBSI"), dibenzyldithiocarbamate de zinc (en abrégé "ZBEC") et les mélanges de ces composés. De préférence, on utilise un accélérateur primaire du type sulfénamide.

La composition de caoutchouc peut se trouver sous la forme crue, c'est-à-dire non vulcanisée. La composition de caoutchouc peut se trouver sous la forme cuite, c'est-à-dire vulcanisée.

De préférence, la composition de caoutchouc peut être utilisée dans le pneumatique sous la forme d'une couche. Par couche, on entend tout élément tridimensionnel, de forme et d'épaisseur quelconques, notamment en feuille, bande ou autre élément de section droite quelconque, par exemple rectangulaire ou triangulaire.

### Composite de caoutchouc selon l'invention

Le composite de caoutchouc est renforcé d'au moins un élément de renfort noyé dans la composition de caoutchouc selon l'invention.

Ce composite de caoutchouc peut être préparé selon un procédé comportant au moins les étapes suivantes :
- au cours d'une première étape, combiner au moins un élément de renfort avec une composition de caoutchouc (ou élastomère, les deux termes sont synonymes) pour former un composite de caoutchouc renforcé de l'élément de renfort;
- puis, au cours d'une deuxième étape, réticuler par cuisson, par exemple par vulcanisation, de préférence sous pression, le composite ainsi formé.

Parmi les éléments de renforts, on pourra citer les éléments de renfort textiles, métalliques ou hybrides textile-métal.

Par textile, on entend, de manière bien connue de l'homme du métier, tout matériau en matière autre que métallique, qu'elle soit naturelle comme synthétique, susceptible d'être transformée en fil, fibre par tout procédé de transformation approprié. On peut citer par exemple, sans que les exemples ci-après soient limitatifs, un procédé de filage de polymère tel que par exemple filage au fondu, filage en solution ou filage de gel.

Ce matériau textile peut consister en un fil ou fibre ou également en un tissu réalisé à partir de fils ou fibres, par exemple d'un tissu tramé avec fils de chaîne et fils de trame, ou encore d'un tissu croisé avec fils croisés.

De préférence, ce matériau textile de l'invention est choisi dans le groupe constitué par les monofilaments (ou fils unitaires), les fibres multifilamentaires, les assemblages de tels fils ou fibres, et les mélanges de tels matériaux. Il s'agit plus particulièrement d'un monofilament, d'une fibre multifilamentaire ou d'un retors.

Par fil ou fibre, on entend de manière générale tout élément longiligne de grande longueur relativement à sa section transversale, quelle que soit la forme de cette dernière, par exemple circulaire, oblongue, rectangulaire ou carrée, ou même plate, ce fil pouvant être rectiligne comme non rectiligne, par exemple torsadé, ou ondulé. La plus grande dimension de sa section transversale est préférentiellement inférieure à 5 mm, plus préférentiellement inférieur à 3 mm.

Ce fil ou cette fibre peut prendre tout forme connue, il peut s'agir par exemple d'un monofilament élémentaire de diamètre important (par exemple et de préférence égal ou supérieur à 50 µm), d'une fibre multifilamentaire (constituée d'une pluralité de filaments élémentaires de faible diamètre, typiquement inférieur à 30 µm), d'un retors ou câblé textile formé de plusieurs fibres ou monofilaments textiles retordus ou câblés ensemble, ou encore d'un assemblage, un groupe, une rangée de fils ou fibres tels que par exemple une bande ou bandelette comportant plusieurs de ces monofilaments, fibres, retors ou câblés regroupés ensemble, par exemple alignés selon une direction principale, rectiligne ou pas.

Les matériaux textiles peuvent être en matière organique ou polymérique, comme en matière inorganique.

A titre d'exemples de matières inorganiques, on citera le verre, le carbone.

L'invention est préférentiellement mise en oeuvre avec des matériaux en matière polymérique, du type thermoplastique comme non thermoplastique.

A titre d'exemples de matières polymériques du type non thermoplastique, on citera par exemple l'aramide (polyamide aromatique) et la cellulose, naturelle comme artificielle, telle que le coton, la rayonne, le lin, le chanvre.

A titre d'exemples de matières polymériques du type thermoplastiques, on citera préférentiellement les polyamides aliphatiques et les polyesters. Parmi les polyamides aliphatiques, on peut citer notamment les polyamides 4-6, 6, 6-6, 11 ou 12. Parmi les polyesters, on peut citer par exemple les PET (polyéthylène téréphthalate), PEN (polyéthylène naphthalate), PBT (polybutylène téréphthalate), PBN (polybutylène naphthalate), PPT (polypropylène téréphthalate), PPN (polypropylène naphthalate).

Par métallique, on entend par définition un ou des éléments filaires constitués majoritairement (c'est-à-dire pour plus de 50% de sa masse) ou intégralement (pour 100% de sa masse) d'un matériau métallique. De préférence, le matériau métallique est l'acier, plus préférentiellement en acier perlitique (ou ferrito-perlitique) au carbone comprenant avantageusement entre 0,4% et 1,2% en masse de carbone.

L'élément de renfort métallique peut être un monofilament, un câble comprenant plusieurs monofilaments métalliques ou un câble multi-torons comprenant plusieurs câbles alors appelés torons.

Dans le cas préféré où l'élément de renfort comprend plusieurs monofilaments métalliques ou plusieurs torons, les monofilaments métalliques ou les torons sont assemblés par retordage ou par câblage. On rappelle qu'il existe deux techniques possibles d'assemblage:
- soit par retordage : les monofilaments métalliques ou les torons subissent à la fois une torsion collective et une torsion individuelle autour de leur propre axe, ce qui génère un couple de détorsion sur chacun des monofilaments ou torons ;
- soit par câblage: les monofilaments métalliques ou les torons ne subissent qu'une torsion collective et ne subissent pas de torsion individuelle autour de leur propre axe.

De façon optionnelle, l'élément de renfort comprend plusieurs monofilaments et est du type gommé in situ, c'est-à-dire que l'élément de renfort est gommé de l'intérieur, pendant sa fabrication même par une gomme de remplissage. De tels éléments filaires métalliques sont connus de l'homme du métier. La composition de la gomme de remplissage peut être identique ou non à la composition de caoutchouc dans laquelle l'élément de renfort est noyé.

Chaque élément de renfort, lorsqu'il est textile, est de préférence revêtu d'une couche d'une composition adhésive ou colle. La colle utilisée est par exemple du type RFL (Résorcinol-Formaldéhyde-Latex) ou par exemple, telle que décrite dans les publications WO2013017421, WO2013017422, WO2013017423 ou encore WO2015007642. Ainsi, la composition de caoutchouc selon l'invention est au contact direct de la composition adhésive. La composition adhésive est interposée entre la composition de caoutchouc selon l'invention et l'élément de renfort.

### Pneumatique selon l'invention

De tels pneumatiques sont par exemple ceux destinés à équiper des véhicules à moteur de type tourisme, SUV (*"Sport Utility Vehicles*"), deux roues (notamment vélos, motos), avions, comme des véhicules industriels choisis parmi camionnettes, "Poids-lourd" - c'est-à-dire métro, bus, engins de transport routier (camions, tracteurs, remorques), véhicules hors-la-route tels qu'engins agricoles ou de génie civil -, autres véhicules de transport ou de manutention.

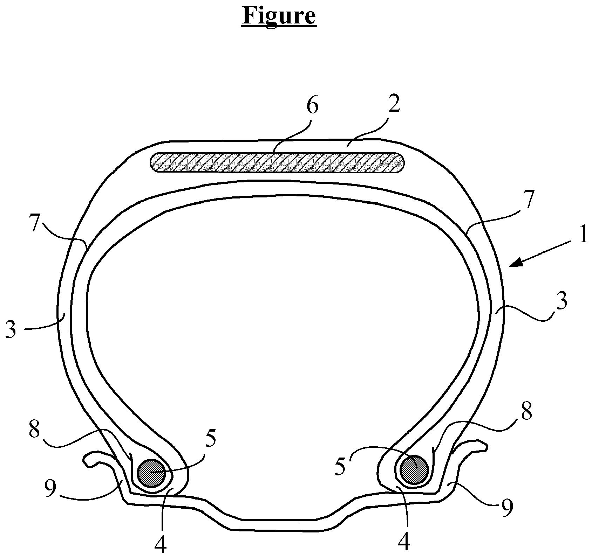

A titre d'exemple, la figure unique annexée représente de manière très schématique (sans respect d'une échelle spécifique), une coupe radiale d'un pneumatique conforme à l'invention pour véhicule du type poids lourd.

Ce pneumatique 1 comporte un sommet 2 renforcé par une armature de sommet ou ceinture 6, deux flancs 3 et deux bourrelets 4, chacun de ces bourrelets 4 étant renforcé avec une tringle 5. Le sommet 2 est surmonté d'une bande de roulement non représentée sur cette figure schématique. Une armature de carcasse 7 est enroulée autour des deux tringles 5 dans chaque bourrelet 4, le retournement 8 de cette armature 7 étant par exemple disposé vers l'extérieur du pneumatique 1 qui est ici représenté monté sur sa jante 9. L'armature de carcasse 7 est de manière connue en soi constituée d'au moins une nappe renforcée par des câbles dits "radiaux", par exemple métalliques, c'est-à-dire que ces câbles sont disposés pratiquement parallèles les uns aux autres et s'étendent d'un bourrelet à l'autre de manière à former un angle compris entre 80° et 90° avec le plan circonférentiel médian (plan perpendiculaire à l'axe de rotation du pneumatique qui est situé à mi-distance des deux bourrelets 4 et passe par le milieu de l'armature de sommet 6).

Ce pneumatique 1 de l'invention a par exemple pour caractéristique qu'au moins une armature de sommet 6 et/ou son armature de carcasse 7 comporte une composition de caoutchouc ou un composite selon l'invention. Bien entendu, l'invention concerne les objets précédemment décrits, à savoir le composite en caoutchouc et le pneumatique, tant à l'état cru (avant cuisson ou vulcanisation) qu'à l'état cuit (après cuisson).

### Procédé selon l'invention

Le procédé de fabrication décrit précédemment et ci-après permet de fabriquer la composition selon l'invention.

La composition de caoutchouc peut être fabriquée dans des mélangeurs appropriés, en utilisant deux phases de préparation successives bien connues de l'homme du métier :
- une première phase de travail ou malaxage thermomécanique (phase dite "non-productive") à haute température, jusqu'à une température maximale comprise entre 110°C et 190°C, de préférence entre 130°C et 180°C,
- suivie d'une seconde phase de travail mécanique (phase dite "productive") jusqu'à une plus basse température, typiquement inférieure à 110°C, par exemple entre 40°C et 100°C, phase de finition au cours de laquelle est incorporé le système de réticulation.

Dans un mode de réalisation, le procédé comporte les étapes suivantes :
- incorporer à un élastomère diénique, au cours d'une première étape (dite "non-productive"), une charge renforçante, en malaxant thermomécaniquement le tout (par exemple en une ou plusieurs fois), jusqu'à atteindre une température maximale comprise entre 110°C et 190°C ;
- refroidir l'ensemble à une température inférieure à 100°C ;
- incorporer ensuite, au cours d'une seconde étape (dite "productive"), un système de réticulation, l'aldéhyde de formule **(A)** et le polyphénol aromatique ;
- malaxer le tout jusqu'à une température maximale inférieure à 110°C.

A titre d'exemple, la phase non-productive est conduite en une seule étape thermomécanique au cours de laquelle on introduit, dans un mélangeur approprié tel qu'un mélangeur interne usuel, dans un premier temps tous les constituants de base nécessaires (un élastomère diénique, charge renforçante,), puis dans un deuxième temps, par exemple après une à deux minutes de malaxage, les autres additifs, éventuels agents de recouvrement de la charge ou de mise en oeuvre complémentaires, à l'exception du système de réticulation. La durée totale du malaxage, dans cette phase non-productive, est de préférence comprise entre 1 et 15 min.

Apres refroidissement du mélange ainsi obtenu, on incorpore alors dans un mélangeur externe tel qu'un mélangeur à cylindres, maintenu à basse température (par exemple entre 40°C et 100°C), le système de réticulation, l'aldéhyde de formule **(A)** et le polyphénol aromatique. L'ensemble est alors mélangé (phase productive) pendant quelques minutes, par exemple entre 2 et 15 min.

La composition finale ainsi obtenue peut ensuite être calandrée, par exemple sous la forme d'une feuille, d'une plaque notamment pour une caractérisation au laboratoire, ou encore extrudée, par exemple pour former un profilé de caoutchouc utilisé pour la fabrication d'un pneumatique.

De façon analogue au composite selon l'invention, le procédé de fabrication du pneumatique comprend :
- l'étape de fabrication de la composition décrite ci-dessus et
- une étape de réticulation de cette composition, par exemple par vulcanisation, de préférence sous pression, pour former le pneumatique selon l'invention.

L'invention ainsi que ses avantages seront aisément compris à la lumière des exemples de réalisation qui suivent.

### Exemples de réalisation de l'invention et essais comparatifs

Ces essais démontrent que :
- la rigidité de la composition de caoutchouc est largement améliorée par rapport à une composition de caoutchouc utilisant une résine renforçante classique à base d'un accepteur de méthylène avec l'HMT ou l'H3M en tant que donneur de méthylène et que
- la rigidité de la composition de caoutchouc est maintenue aux températures élevées, en particulier pour des températures allant jusqu'à 150°C.
En outre, la résine phénol-aldéhyde de la composition selon l'invention est dépourvue de formaldéhyde et n'en génère pas lors de sa formation.

Pour cela, plusieurs compositions de caoutchouc, notées ci-après T0 à T14 et 15 ont été préparées comme indiqué précédemment et sont rassemblées dans le tableau 1 annexé ci-après.

Toutes les compositions T0 à T14 et 15 ont une partie commune dans leurs formulations (exprimées en pce, parties en poids pour cent parties d'élastomère): 100 pce de caoutchouc naturel, 75 pce de noir de carbone N326, 1,5 pce de N-1, 3-diméthylbutyl-N-phényl-para-phénylènediamine, 1,5 pce d'acide stéarique, 5 pce de ZnO, 1 pce de N-tertiarybutyl-2-benzothiazole sulfamide et 2,5 pce de soufre insoluble 20H.

La composition T0 ne comprend aucune résine renforçante ajoutée à cette partie commune.

En plus de la partie commune, la composition T1 comprend une résine renforçante à base d'hexa-méthylènetétramine (1,6 pce) et d'une résine phénolique pré-condensée (4 pce). La composition T1 représente une composition classique de l'état de la technique présentant une rigidité supérieure à celle de la composition T0.

En plus de la partie commune, chaque composition T2 à T7 comprend 14 pce de phénol et 14 pce de l'aldéhyde indiqués dans le tableau 1.

En plus de la partie commune, chaque composition T8 à T14 comprend 14 pce de polyphénol aromatique et 14 pce du polyaldéhyde aromatique indiqués dans le tableau 1.

En plus de la partie commune, la composition 15 comprend 14 pce de polyphénol aromatique et 14 pce de l'aldéhyde de formule (A) indiqués dans le tableau 1.

Les compositions T0 à T14 ne sont pas conformes à l'invention contrairement à la composition 15 qui est conforme à l'invention.

La composition de caoutchouc 15 selon l'invention comprend une résine phénol-aldéhyde à base :
- d'au moins un polyphénol aromatique comprenant au moins un noyau aromatique porteur d'au moins deux fonctions hydroxyle en position méta l'une par rapport à l'autre, les deux positions ortho d'au moins une des fonctions hydroxyle étant non substituées, et
- d'au moins un aldéhyde de formule **(A).**

Chaque polyphénol aromatique de la résine de chaque composition T8 à T14 et 15 selon l'invention est choisi dans le groupe constitué par le résorcinol, le phloroglucinol, le 2,2',4,4'-tétrahydroxydiphényl sulfide, la 2,2',4,4'-tétrahydroxybenzophenone, les résines pré-condensées à partir de ces phénols et les mélanges de ces composés.

Chaque polyphénol aromatique de chaque composition T8 et T9 comprend un unique noyau aromatique, ici benzénique, porteur de deux, et seulement deux, fonctions hydroxyle en position méta l'une par rapport à l'autre. En l'espèce, il s'agit du résorcinol.

Chaque polyphénol de chaque composition T10 et T11 et 15 selon l'invention comprend un unique noyau aromatique, ici benzénique, porteur de trois, et seulement trois, fonctions hydroxyle en position méta l'une par rapport à l'autre. En l'espèce, il s'agit du phloroglucinol.

Pour les polyphénols aromatiques de chaque composition T8 à T11 et 15 selon l'invention, le reste du noyau aromatique du polyphénol aromatique est non substitué. En particulier, les deux positions ortho de chaque fonction hydroxyle sont non substituées.

Chaque polyphénol aromatique de chaque composition T12 à T14 comprend plusieurs noyaux aromatiques, ici benzéniques, au moins deux d'entre eux étant chacun porteur d'au moins deux fonctions hydroxyle en position méta l'une par rapport à l'autre. Les deux positions ortho d'au moins une des fonctions hydroxyle de chaque noyau aromatique sont non substituées.

Le polyphénol aromatique de la composition T14 est une résine pré-condensée à partir du résorcinol et du formaldéhyde.

En variante, dans la composition selon l'invention, on pourrait exploiter, en remplacement du phloroglucinol, un polyphénol aromatique comprenant un unique noyau aromatique, par exemple benzénique, porteur de deux, et seulement deux, fonctions hydroxyle en position méta l'une par rapport à l'autre. Par exemple, le résorcinol comme dans les compositions T8 et T9.

Dans une autre variante, dans la composition selon l'invention, on pourrait exploiter, en remplacement du phloroglucinol, un polyphénol aromatique comprenant plusieurs noyaux aromatiques, par exemple benzéniques, au moins deux d'entre eux étant chacun porteur d'au moins deux fonctions hydroxyle en position méta l'une par rapport à l'autre, comme dans les compositions T12 et T13. Les deux positions ortho d'au moins une des fonctions hydroxyle de chaque noyau aromatique sont non substituées.

Dans encore une autre variante, dans la composition selon l'invention, on pourrait exploiter, en remplacement du phloroglucinol, un polyphénol aromatique comprenant une résine pré-condensée à partir du résorcinol et du formaldéhyde comme dans la composition T14.

Chaque polyaldéhyde aromatique de chaque composition T8 à T14 est soit le 1,3-benzène-dicarboxaldéhyde, soit le 1,4-benzène-dicarboxaldéhyde. En variante, cela pourrait être un mélange du 1,3-benzène-dicarboxaldéhyde et du 1,4-benzènedicarboxaldéhyde.

L'aldéhyde de formule **(A)** de la composition 15 selon l'invention est choisi dans le groupe constitué par le furfuraldéhyde, le 2,5-furanedicarboxaldéhyde et les mélanges de ces composés. Ici, l'aldéhyde de formule **(A)** est le 2,5-furanedicarboxaldéhyde.

On a préparé les compositions T1 à T14 et 15 conformément au procédé précédemment décrit, puis on a caractérisé ces compositions à l'aide de plusieurs tests de caractérisation décrits ci-dessous.

On a tout d'abord réalisé une caractérisation de la rigidité à haute température dans laquelle on a chauffé le mélange à 150°C jusqu'à l'obtention du couple rhéométrique maximum.

Une fois vulcanisée, on a réalisé une caractérisation de la rigidité à 23°C de la composition lors d'un essai en traction.

### Caractérisation de la rigidité à haute température - Couple rhéométrique maximum

Les mesures sont effectuées à 150°C avec un rhéomètre à chambre oscillante, selon la norme DIN 53529 - partie 3 (juin 1983). L'évolution du couple rhéométrique en fonction du temps décrit l'évolution de la rigidification de la composition par suite de la vulcanisation et de la réticulation de la résine phénol-aldéhyde. De l'évolution du couple rhéométrique, on détermine le couple rhéométrique maximum Cmax reporté dans le tableau 1. Plus le couple rhéométrique maximum Cmax est élevé, plus la composition présente une rigidité pouvant être maintenue à haute température.

### Caractérisation de la rigidité à 23°C - Essai de traction

Ces essais permettent de déterminer les contraintes d'élasticité et les propriétés à la rupture. Sauf indication différente, ils sont effectués conformément à la norme ASTM D 412 de 1998 (éprouvette C). On mesure en seconde élongation (i.e. après un cycle d'accommodation) les modules sécants dits "nominaux" (ou contraintes apparentes, en MPa) à 10% d'allongement (noté "MA10"). Toutes ces mesures de traction sont effectuées dans les conditions normales de température et d'hygrométrie, selon la norme ASTM D 1349 de 1999 et reportées dans le tableau 1.

Tout d'abord, les résultats du tableau 1 montre que l'utilisation d'une résine renforçante de l'état de la technique (T1) permet d'obtenir une rigidité à 23°C et une tenue de cette rigidité à des températures plus élevées meilleures que pour une composition dépourvue de résine renforçante (T0). Toutefois, malgré une tenue de la rigidité en température supérieure à celles des compositions T2 à T7, la rigidité à 23°C de la composition T1 est bien inférieure à celle de chaque composition T8 à T14 et 15 selon l'invention.

De plus, les résultats du tableau 1 montrent que l'utilisation d'un monophénol (T2) aromatique ne permet pas d'obtenir une rigidité à 23°C suffisante, ni une tenue de cette rigidité à des températures plus élevées contrairement aux polyphénols aromatiques des compositions T8 à T14 et 15 conforme à l'invention.

En outre, les résultats du tableau 1 montrent que l'utilisation d'un aldéhyde aromatique comprenant un noyau benzénique portant une unique fonction aldéhyde (T3 et T4) ne permet pas d'obtenir une meilleure rigidité à 23°C par rapport à la composition T1, ni que cette rigidité puisse être maintenue à des températures plus élevées contrairement à l'aldéhyde de formule **(A)** de la composition 15. Ces résultats sont relativement inattendus pour l'homme du métier compte tenu du fait que, *a priori,* l'aldéhyde de formule **(A)** de la composition 15 présente une réactivité inférieure à celle du monoaldéhyde aromatique des compositions T3 et T4.

Les résultats du tableau 1 montrent également que l'utilisation du 1,2-benzène-dicarboxaldéhyde (T5), si elle permet l'obtention d'une rigidité à 23°C améliorée par rapport à la composition T1 ne permet pas de maintenir cette rigidité à des températures élevées contrairement à l'aldéhyde de formule **(A)** de la composition 15.

L'utilisation d'un polyphénol aromatique dont les deux fonctions hydroxyle sont en positions para (T6) l'une par rapport à l'autre ne permet pas d'obtenir une rigidité à 23°C améliorée par rapport à la composition T1. De plus, un tel polyphénol ne permet pas le maintien de cette rigidité à des températures plus élevées.

Enfin, les résultats du tableau 1 montrent que l'utilisation d'un polyphénol aromatique dont les deux fonctions hydroxyle sont en positions ortho (T7) l'une par rapport à l'autre permet effectivement l'obtention d'une rigidité à 23°C améliorée par rapport à la composition T1 sans toutefois permettre une tenue satisfaisante de cette rigidité aux températures élevées contrairement aux polyphénols aromatiques conformes à l'invention (T8 à T14 et 15 conforme à l'invention).

L'invention ne se limite pas aux modes de réalisation décrits précédemment.

**Tableau 1**

| **Composition** | **Donneur de méthyléne** | **Phénol** | **MA10 (MPa)** | **Cmax (dN.m)** |
|---|---|---|---|---|
| T0 | / | / | 7,2 | 16 |
| T1 | Hexa-méthylènetétramine (1) | Résine SRF (2) | 16,5 | 43 |

| **Composition** | **Aldéhyde** | **Phénol** | **MA10 (MPa)** | **Cmax (dN.m)** |
|---|---|---|---|---|
| T2 | 1,4-benzène-dicarboxaldéhyde (5) | 3-tert-butylphénol (7) | 4,7 | 7 |
| T3 | Benzaldéhyde (6) | Phloroglucinol (11) | 14,7 | 14 |
| T4 | Benzaldéhyde (6) | Résorcinol (9) | 12,1 | 7 |
| T5 | 1,2-benzène-dicarboxaldéhyde (3) | Résorcinol (9) | 21,5 | 20 |
| T6 | 1,4-benzène-dicarboxaldéhyde (5) | Hydroquinone (10) | 13,6 | 7 |
| T7 | 1,4-benzène-dicarboxaldéhyde (5) | 1,2-dihydroxybenzène (8) | 36,2 | 25 |

| **Composition** | **Polyaldéhyde aromatique** | **Polyphénol aromatique** | **MA10 (MPa)** | **Cmax (dN.m)** |
|---|---|---|---|---|
| T8 | 1,3-benzène-dicarboxaldéhyde (4) | Résorcinol (9) | 28,7 | 30 |
| T9 | 1,4-benzène-dicarboxaldéhyde (5) | Résorcinol (9) | 32 | 73 |
| T10 | 1 ,3-benzène-dicarboxaldéhyde (4) | Phloroglucinol (11) | 39 | 35 |
| T11 | 1 ,4-benzène-dicarboxaldéhyde (5) | Phloroglucinol (11) | 33 | 30 |
| T12 | 1,4-benzène-dicarboxaldéhyde (5) | 2,2',4,4'-tétrahydroxydiphényl sulfide (12) | 32 | 45 |
| T13 | 1,4-benzène-dicarboxaldéhyde (5) | 2,2',4,4'-tétrahydroxybenzophenone (13) | 36,1 | 50 |
| T14 | 1,4-benzène-dicarboxaldéhyde (5) | Résine SRF (2) | 35,1 | 50 |

| **Composition** | **Aldéhyde de formule (A)** | **Polyphénol aromatique** | **MA10 (MPa)** | **Cmax (dN.m)** |
|---|---|---|---|---|
| 15 | 2,5-furanedicarboxaldéhyde (14) | Phloroglucinol (11) | 25,7 | 40 |

| | | | | |
|---|---|---|---|---|
| (1) Hexa-méthylènetétramine (de la société Sigma-Aldrich ; de pureté ≥ 99%) ; (2) Résine précondensée SRF 1524 (de la société Schenectady ; diluée à 75%); (3) 1,2-benzènedicarboxaldéhyde (de la société ABCR ; de pureté 98%) ; (4) 1,3-benzènedicarboxaldéhyde (de la société ABCR ; de pureté 98%) ; (5) 1,4-benzènedicarboxaldéhyde (de la société ABCR ; de pureté 98%) ; (6) Benzaldhéhyde (de la société Sigma-Aldrich, de pureté ≥ 99,5%) ; (7) 3-tert-butylphénol (de la société Sigma-Aldrich, de pureté 99 %) ; (8) 1,2-dihydroxybenzène (de la société Sigma-Aldrich, de pureté 99 %) ; (9) Résorcinol (de la société Sumitomo ; de pureté 99.5%) ; (10) Hydroquinone (de la société Sigma-Aldrich, de pureté 99 %) ; (11) Phloroglucinol (de la société Alfa Aesar ; de pureté 99%) ; (12) 2,2',4,4'-tétrahydroxydiphényl sulfide (de la société Alfa Aesar ; de pureté 98%) ; (13) 2,2',4,4'-tétrahydroxybenzophenone (de la société Sigma-Aldrich, de pureté 97 %) ; (14) 2,5-furanedicarboxaldéhyde (de la société Aldrich ; de pureté 97%). | | | | |

## Revendications

1. Composition de caoutchouc pouvant être calandrée ou extrudée, **caractérisé en ce qu'**elle comprend donc au moins une résine renforçante réticulée, cette résine renforçante étant constituée par une résine phénol-aldéhyde comportant le produit de la réaction entre au moins :
- un polyphénol aromatique comprenant au moins un noyau aromatique porteur d'au moins deux fonctions hydroxyle en position méta l'une par rapport à l'autre, les deux positions ortho d'au moins une des fonctions hydroxyle étant non substituées, et
- un aldéhyde de formule **(A)** : 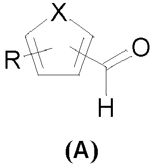 dans laquelle :
X comprend N, S ou O
R représente -H ou -CHO
dans laquelle la quantité d'aldéhyde de formule (A) va de 0,1 à 30 pce et la quantité de polyphénol aromatique va de 0,1 à 30 pce ; et
dans laquelle la composition comprend au moins un élastomère ou un et au moins un autre composant avec la composition de caoutchouc étant dans un état plastique à l'état cru (non-réticulé) et dans un état élastique à l'état cuit (réticulé) mais en aucun cas dans un état liquide .

2. Composition de caoutchouc selon la revendication précédente, dans laquelle le noyau aromatique du polyphénol aromatique est porteur de trois fonctions hydroxyle en position méta les unes par rapport aux autres.

3. Composition de caoutchouc selon la revendication 1 ou 2, dans laquelle les deux positions ortho de chaque fonction hydroxyle sont non substituées.

4. Composition de caoutchouc selon l'une quelconque des revendications 1 à 3, dans laquelle le reste du noyau aromatique du polyphénol aromatique est non substitué.

5. Composition de caoutchouc selon l'une quelconque des revendications précédentes, dans laquelle le polyphénol aromatique comprend plusieurs noyaux aromatiques, au moins deux d'entre eux étant chacun porteur d'au moins deux fonctions hydroxyle en position méta l'une par rapport à l'autre, les deux positions ortho d'au moins une des fonctions hydroxyle d'au moins un noyau aromatique étant non substituées.

6. Composition de caoutchouc selon la revendication précédente, dans laquelle au moins un des noyaux aromatiques du polyphénol aromatique est porteur de trois fonctions hydroxyle en position méta les unes par rapport aux autres.

7. Composition de caoutchouc selon la revendication 5 ou 6, dans laquelle les deux positions ortho de chaque fonction hydroxyle d'au moins un noyau aromatique sont non substituées.

8. Composition de caoutchouc selon l'une quelconque des revendications 5 à 7, dans laquelle les deux positions ortho de chaque fonction hydroxyle de chaque noyau aromatique sont non substituées.

9. Composition de caoutchouc selon l'une quelconque des revendications précédentes, dans lequel le ou chaque noyau aromatique du polyphénol aromatique est un noyau benzénique.

10. Composition de caoutchouc selon l'une quelconque des revendications précédentes, dans laquelle le polyphénol aromatique est choisi dans le groupe constitué par le résorcinol, le phloroglucinol, le 2,2',4,4'-tétrahydroxydiphényl sulfide, la 2,2',4,4'-tétrahydroxybenzophénone, les résines pré-condensées à partir d'au moins un de ces phénols et les mélanges de ces composés.

11. Composition de caoutchouc selon l'une quelconque des revendications précédentes, dans laquelle l'aldéhyde est de formule générale **(A'):** 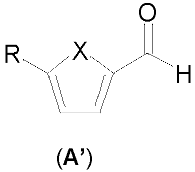 dans laquelle X représente NH, S ou O et R représente -H ou -CHO.

12. Composition de caoutchouc selon l'une quelconque des revendications précédentes, dans lequel l'aldéhyde est choisi dans le groupe constitué par le furfuraldéhyde, le 2,5-furanedicarboxaldéhyde et les mélanges de ces composés.

13. Composition de caoutchouc selon l'une quelconque des revendications précédentes, comprenant un élastomère diénique choisi dans le groupe constitué par les polybutadiènes, les polyisoprènes de synthèse, le caoutchouc naturel, les copolymères de butadiène, les copolymères d'isoprène et les mélanges de ces élastomères.

14. Procédé de fabrication d'une composition de caoutchouc pouvant être calandrée ou extrudée, **caractérisé en ce qu'**il comprend une étape de mélangeage :
- d'au moins un élastomère,
- au moins une résine renforçante réticulée, cette résine renforçante étant constituée par une résine phénol-aldéhyde comportant le produit de la réaction entre au moins :
- un polyphénol aromatique comprenant au moins un noyau aromatique porteur d'au moins deux fonctions hydroxyle en position méta l'une par rapport à l'autre, les deux positions ortho d'au moins une des fonctions hydroxyle étant non substituées, et
- un aldéhyde de formule **(A)** : 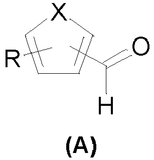 dans laquelle :
X comprend N, S ou O
R représente -H ou -CHO ;
dans lequel la quantité d'aldéhyde de formule (A) va de 0,1 à 30 pce et la quantité de polyphénol aromatique va de 0,1 à 30 pce ; et
dans lequel la composition comprend au moins un élastomère ou un et au moins un autre composant avec la composition de caoutchouc étant dans un état plastique à l'état cru (non-réticulé) et dans un état élastique à l'état cuit (réticulé) mais en aucun cas dans un état liquide.

15. Procédé selon la revendication précédente, comprenant les étapes suivantes :
- incorporer à un élastomère diénique, au cours d'une première étape, une charge renforçante, en malaxant thermomécaniquement le tout, jusqu'à atteindre une température maximale comprise entre 110°C et 190°C ;
- refroidir l'ensemble à une température inférieure à 100°C ;
- incorporer ensuite, au cours d'une seconde étape, un système de réticulation, le polyphénol aromatique et l'aldéhyde de formule **(A);**
- malaxer le tout jusqu'à une température maximale inférieure à 110°C.

16. Composite de caoutchouc renforcé d'au moins un élément de renfort noyé dans une composition de caoutchouc, **caractérisé en ce que** la composition de caoutchouc est selon l'une quelconque des revendications 1 à 13.

17. Pneumatique (1), **caractérisé en ce qu'**il comprend une composition de caoutchouc selon l'une quelconque des revendications 1 à 13 ou un composite de caoutchouc selon la revendication précédente.

## Patentansprüche

1. Kautschukzusammensetzung, die kalandriert oder extrudiert werden kann, **dadurch gekennzeichnet, dass** sie daher mindestens ein vernetztes verstärkendes Harz umfasst, wobei dieses verstärkende Harz aus mindestens einem Phenol-Aldehyd-Harz besteht, das das Produkt der Reaktion zwischen mindestens:
- einem aromatischen Polyphenol mit mindestens einem aromatischen Kern, der mindestens zwei Hydroxylfunktionen in meta-Position zueinander trägt, wobei die beiden ortho-Positionen mindestens einer der Hydroxylfunktionen unsubstituiert sind, und
- einem Aldehyd der Formel (A): 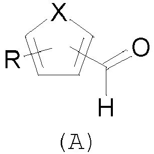 in der:
X N, S oder 0 umfasst,
R für -H oder -CHO steht,
umfasst, wobei die Menge an Aldehyd der Formel (A) im Bereich von 0,1 bis 30 phe liegt und die Menge an aromatischem Polyphenol im Bereich von 0,1 bis 30 phe liegt; und
wobei die Zusammensetzung mindestens ein Elastomer oder einen und mindestens einen weiteren Bestandteil umfasst, wobei nun die Kautschukzusammensetzung im ungehärteten (unvernetzten) Zustand in einem plastischen Zustand und im gehärteten (vernetzten) Zustand in einem elastischen Zustand, aber in keinem Fall in einem flüssigen Zustand vorliegt.

2. Kautschukzusammensetzung nach dem vorhergehenden Anspruch, wobei der aromatische Kern des aromatischen Polyphenols drei Hydroxylfunktionen in meta-Position zueinander trägt.

3. Kautschukzusammensetzung nach Anspruch 1 oder 2, wobei die beiden ortho-Positionen jeder Hydroxylfunktion unsubstituiert sind.

4. Kautschukzusammensetzung nach einem der Ansprüche 1 bis 3, wobei der aromatische Kern des aromatischen Polyphenols unsubstituiert ist.

5. Kautschukzusammensetzung nach einem der vorhergehenden Ansprüche, wobei das aromatische Polyphenol mehrere aromatische Kerne umfasst, von denen mindestens zwei jeweils mindestens zwei Hydroxylfunktionen in meta-Position zueinander tragen, wobei die beiden ortho-Positionen mindestens einer der Hydroxylfunktionen mindestens eines aromatischen Kerns unsubstituiert sind.

6. Kautschukzusammensetzung nach dem vorhergehenden Anspruch, wobei mindestens einer der aromatischen Kerne des aromatischen Polyphenols drei Hydroxylfunktionen in meta-Position zueinander trägt.

7. Kautschukzusammensetzung nach Anspruch 5 oder 6, wobei die beiden ortho-Positionen jeder Hydroxylfunktion mindestens eines aromatischen Kerns unsubstituiert sind.

8. Kautschukzusammensetzung nach einem der Ansprüche 5 bis 7, wobei die beiden ortho-Positionen jeder Hydroxylfunktion jedes aromatischen Kerns unsubstituiert sind.

9. Kautschukzusammensetzung nach einem der vorhergehenden Ansprüche, wobei der bzw. jeder aromatische Kern des aromatischen Polyphenols ein Benzolkern ist.

10. Kautschukzusammensetzung nach einem der vorhergehenden Ansprüche, wobei das aromatische Polyphenol aus der Gruppe bestehend aus Resorcinol, Phloroglucinol, 2,2',4,4'-Tetrahydroxydiphenylsulfid, 2,2',4,4'-Tetrahydroxybenzophenon, vorkondensierten Harzen mindestens eines dieser Phenole und Mischungen dieser Verbindungen ausgewählt ist.

11. Kautschukzusammensetzung nach einem der vorhergehenden Ansprüche, wobei der Aldehyd die allgemeine Formel (A') aufweist: 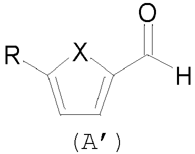 in der X für NH, S oder 0 steht und R für -H oder -CHO steht.

12. Kautschukzusammensetzung nach einem der vorhergehenden Ansprüche, wobei der Aldehyd aus der Gruppe bestehend aus Furfuraldehyd, 2,5-Furandicarboxaldehyd und Mischungen dieser Verbindungen ausgewählt ist.

13. Kautschukzusammensetzung nach einem der vorhergehenden Ansprüche, umfassend ein Dienelastomer ausgewählt aus der Gruppe bestehend aus Polybutadienen, synthetischen Polyisoprenen, Naturkautschuk, Butadien-Copolymeren, Isopren-Copolymeren und Mischungen dieser Elastomere.

14. Verfahren zur Herstellung einer Kautschukzusammensetzung, die kalandriert oder extrudiert werden kann, **dadurch gekennzeichnet, dass** es einen Schritt des Mischens von
- mindestens einem Elastomer,
- mindestens einem vernetzten verstärkenden Harz umfasst, wobei dieses verstärkende Harz aus einem Phenol-Aldehyd-Harz besteht, das das Produkt der Reaktion zwischen mindestens:
- einem aromatischen Polyphenol mit mindestens einem aromatischen Kern, der mindestens zwei Hydroxylfunktionen in meta-Position zueinander trägt, wobei die beiden ortho-Positionen mindestens einer der Hydroxylfunktionen unsubstituiert sind, und
- einem Aldehyd der Formel (A): 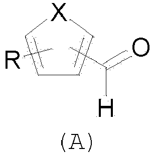 in der:
X N, S oder 0 umfasst,
R für -H oder -CHO steht;
umfasst, wobei die Menge an Aldehyd der Formel (A) im Bereich von 0,1 bis 30 phe liegt und die Menge an aromatischem Polyphenol im Bereich von 0,1 bis 30 phe liegt; und
wobei die Zusammensetzung mindestens ein Elastomer oder einen und mindestens einen weiteren Bestandteil umfasst, wobei nun die Kautschukzusammensetzung im ungehärteten (unvernetzten) Zustand in einem plastischen Zustand und im gehärteten (vernetzten) Zustand in einem elastischen Zustand, aber in keinem Fall in einem flüssigen Zustand vorliegt.

15. Verfahren nach dem vorhergehenden Anspruch, das die folgenden Schritte umfasst:
- Einarbeiten eines verstärkenden Füllstoffs in ein Dienelastomer im Lauf eines ersten Schritts, indem man das Ganze thermomechanisch knetet, bis eine Höchsttemperatur zwischen 110 °C und 190 °C erreicht ist;
- Abkühlen des Ganzen auf eine Temperatur unter 100 °C;
- dann Einarbeiten eines Vernetzungssystems, des aromatischen Polyphenols und des Aldehyds der Formel (A) im Lauf eines zweiten Schritts;
- Kneten des Ganzen bis zu einer maximalen Temperatur von weniger als 110 °C.

16. Kautschukverbundwerkstoff, verstärkt mit mindestens einem in eine Kautschukzusammensetzung eingebetteten Verstärkungselement, **dadurch gekennzeichnet, dass** es sich bei der Kautschukzusammensetzung um eine Kautschukzusammensetzung nach einem der Ansprüche 1 bis 13 handelt.

17. Reifen (1), **dadurch gekennzeichnet, dass** er eine Kautschukzusammensetzung nach einem der Ansprüche 1 bis 13 oder einen Kautschukverbundwerkstoff nach dem vorhergehenden Anspruch umfasst.

## Claims

1. Rubber composition, **characterized in that** it comprises at least one phenol/aldehyde resin based on :
- at least one aromatic polyphenol comprising at least one aromatic ring bearing at least two hydroxyl functions in the meta position relative to one another, the two positions ortho to at least one of the hydroxyl functions being unsubstituted, and
- at least one aldehyde of formula (A): 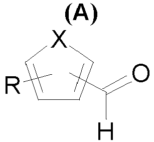 in which:
X comprises N, S or O,
R represents -H or -CHO;
in which the amount of aldehyde of formula (A) is ranging from 0.1 to 30 phr and the amount of aromatic polyphenol is ranging from 0.1 to 30 phr; and
in which the composition comprises at least one elastomer and at least one other component with the rubber composition is in a plastic state in the uncured (non-crosslinked) state and in an elastic state in the cured (crosslinked) state, but never in a liquid state.

2. Rubber composition according to the preceding claim, in which the aromatic ring of the aromatic polyphenol bears three hydroxyl functions in the meta position relative to one another.

3. Rubber composition according to Claim 1 or 2, in which the two positions ortho to each hydroxyl function are unsubstituted.

4. Rubber composition according to any one of Claims 1 to 3, in which the remainder of the aromatic ring of the aromatic polyphenol is unsubstituted.

5. Rubber compositions according to any one of the preceding claims, in which the aromatic polyphenol comprises several aromatic rings, at least two of these each bearing at least two hydroxyl functions in the meta position relative to one another, the two positions ortho to at least one of the hydroxyl functions of at least one aromatic ring being unsubstituted.

6. Rubber composition according to the preceding claim, in which at least one of the aromatic rings of the aromatic polyphenol bears three hydroxyl functions in the meta position relative to one another.

7. Rubber composition according to Claim 5 or 6, in which the two positions ortho to each hydroxyl function of at least one aromatic ring are unsubstituted.

8. Rubber composition according to any one of Claims 5 to 7, in which the two positions ortho to each hydroxyl function of each aromatic ring are unsubstituted.

9. Rubber composition according to any one of the preceding claims, in which the, or each, aromatic ring of the aromatic polyphenol is a benzene ring.

10. Rubber composition according to any one of the preceding claims, in which the aromatic polyphenol is selected from the group consisting of resorcinol, phloroglucinol, 2,2',4,4'-tetrahydroxydiphenyl sulphide, 2,2',4,4'-tetrahydroxybenzophenone, resins precondensed from at least one of these phenols and the mixtures of these compounds.

11. Rubber composition according to any one of the preceding claims, in which the aldehyde is of general formula (A'): 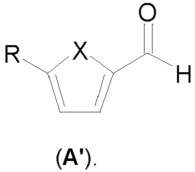 in which X represents NH, S or O and R represents -H or -CHO.

12. Rubber composition according to any one of the preceding claims, in which the aldehyde is selected from the group consisting of furfuraldehyde, 2,5-furandicarboxaldehyde and the mixtures of these compounds.

13. Rubber composition according to any one of the preceding claims, comprising a diene elastomer selected from the group consisting of polybutadienes, synthetic polyisoprenes, natural rubber, butadiene copolymers, isoprene copolymers and the mixtures of these elastomers.

14. Method for manufacturing a rubber composition, **characterized in that** it comprises a step of mixing:
- at least one elastomer,
- at least one aromatic polyphenol comprising at least one aromatic ring bearing at least two hydroxyl functions in the meta position relative to one another, the two positions ortho to at least one of the hydroxyl functions being unsubstituted, and
- at least one aldehyde of formula **(A):** 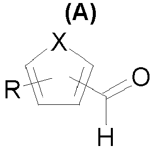 in which:
X comprises N, S or O,
R represents -H or -CHO;
in which the amount of aldehyde of formula (A) is ranging from 0.1 to 30 phr and the amount of aromatic polyphenol is ranging from 0.1 to 30 phr; and
in which the composition comprises at least one elastomer and at least one other component with the rubber composition is in a plastic state in the uncured (non-crosslinked) state and in an elastic state in the cured (crosslinked) state, but never in a liquid state.

15. Method according to the preceding claim, comprising the following steps:
- incorporating, in a diene elastomer, during a first step, a reinforcing filler, everything being kneaded thermomechanically until a maximum temperature of between 110°C and 190°C is reached;
- cooling the combined mixture to a temperature of less than 100°C;
- then incorporating, during a second step, a crosslinking system, the aromatic polyphenol and the aldehyde of formula **(A);**
- kneading everything up to a maximum temperature of less than 110°C.

16. Rubber composite reinforced with at least one reinforcing element embedded in a rubber composition, **characterized in that** the rubber composition is according to any one of Claims 1 to 13.

17. Tyre (1), **characterized in that** it comprises a rubber composition according to any one of Claims 1 to 13 or a rubber composite according to the preceding claim.
